# EUROPÄISCHE PATENTSCHRIFT

(11) **EP 3 601 211 B1**
(45) Veröffentlichungstag und Bekanntmachung des Hinweises auf die Patenterteilung: **06.01.2021**
(21) Anmeldenummer: 18709303.4
(22) Anmeldetag: 28.02.2018
(51) Int. Cl.: C07C 213/08, C07C 215/10

(54) **KATALYTISCHES VERFAHREN ZUR HERSTELLUNG VON N,N-DIMETHYLGLUCAMIN AUSGEHEND AUS N-METHYLGLUCAMIN**
CATALYTIC PROCESS FOR THE MANUFACTURE OF N,N-DIMETHYLGLUCAMIN DEPARTING FROM N-METHYLGLUCAMIN
PROCEDE CATALYTIQUE POUR LA PREPARATION DE N,N-DIMETHYLGLUCAMINE EN UTILISANT N-METHYLGLUCAMINE

(30) Priorität: 20.03.2017 EP 17161762
(43) Veröffentlichungstag der Anmeldung: 05.02.2020
(73) Patentinhaber: Clariant International Ltd, 4132 Muttenz (CH)
(72) Erfinder: AKGÜN, Ertan, 85049 Ingolstadt (DE); LINK, Martin, 84453 Mühldorf (DE); WERNER, Sarah, 84453 Mühldorf (DE); RAAB, Klaus, 84508 Burgkirchen (DE); KLUG, Peter, 63762 Grossostheim (DE); SCHEITZENEDER, Karl, 84549 Engelsberg (DE); KREUZPOINTNER, Stefan, 84513 Töging (DE)
(74) Vertreter: Mikulecky, Klaus
(86) Internationale Anmeldenummer: PCT/EP2018/054857
(87) Internationale Veröffentlichungsnummer: WO 2018/172026

(56) Entgegenhaltungen:
- EP-A1- 0 614 881
- EP-A1- 0 663 389
- WO-A1-2007/137990
- DE-A1- 2 118 283
- GB-A- 908 203
- US-A- 4 275 238

## Beschreibung

Das hier beschriebene N,N-Dimethylglucamin(D-Glucitol, 1-deoxy-1-(dimethylamino))-ist ein wertvolle Verbindung, welche für eine Vielzahl von kommerziellen Anwendungen geeignet ist. Darunter fällt zum Beispiel der Einsatz als Zusatz in Benetzungsmitteln, Reinigungsmitteln, Weichmachern, Gleit-und Schmiermitteln und Ähnlichem. Neuere Anwendungsgebiete finden sich auch in der Farben- und Lackherstellung, dem Pflanzenschutz, in der Medizin und in der Kosmetik. Das auf nachwachsenden Rohstoffen basierende Produkt kann auch als mildes Neutralisationsmittel oder in protonierter Form als hydrophiles Kation verwendet werden. Für dieses breite Anwendungsspektrum müssen zahlreiche Qualitätsanforderungen eingehalten werden, welche in den bisher beschriebenen Verfahren zur Herstellung von N,N-Dimethylglucamin nicht beschrieben sind.

Das hier beschriebene Verfahren ermöglicht die Herstellung von N,N-Dimethylglucamin- Lösungen aus kommerziell gut zugänglichem N-Methylglucamin bei besonders milden Bedingungen mit Hilfe eines leicht handhabbaren einstufigen Verfahrens unter Einhaltung der für die Anwendung notwendigen Qualitätsanforderungen.

Zur Herstellung von N,N-Dimethylglucamin sind in der Literatur einige Verfahren beschrieben, allerdings liefern diese einen oder mehrere Nachteile.

EP-0614881 beschreibt die Umsetzung von N-Monoalkylpolyhydroxyverbindung mit Aldehyden im Verhältnis 1 : 0,9 - 1,5 bzw. 1 : 1,1 - 1,03 mit anschließender Hydrierung über einem metallischen Hydrierkatalysator, z. B. Raney-Ni zum tertiären Dialkylpolyhdroxyamin. Diese erfordert einen Wasserstoffdruck von 10 bis 150 bar und Temperaturen von 70 bis 150 °C. Insbesondere wird die Umsetzung in zwei Reaktionsschritten durchgeführt, wobei man
a) zunächst ein sekundäres N-Monoalkylpolyhydroxyamin (z. B. N-Methylglucamin) mit einem Aldehyd (z. B. Formaldehyd) im Molverhältnis von 1 : 0,90 bis 1,5, vorzugsweise 1 : 1,03 bis 1,1, in Wasser als Lösungsmittel bei einer Temperatur von 15 bis 60 °C, vorzugsweise von 20 bis 40 °C, und Atmosphärendruck zum N-Monoalykipolyhydroxyamin/Aldehyd-Addukt umsetzt und
b) das im Schritt a) enthaltene Reaktionsprodukt (das im Wesentlichen aus dem gebildeten Addukt und Wasser besteht) in Gegenwart eines metallischen Hydrierkatalysators mit Wasserstoff bei einem Druck von 10 bis 150 bar, vorzugsweise 30 bis 100 bar, und einer Temperatur von 70 bis 150 °C, vorzugsweise 80 bis 130 °C zum tertiären Dialkylpolyhydroxyamin (z. B. N,N-Dimethylglucamin) hydriert.

EP-0614881 beschreibt, dass mit diesem Verfahren hohe Ausbeuten erzielt werden können, allerdings sind keine weiteren Angaben zur Aufarbeitung der rohen Reaktionslösung beschrieben. Die Patentschrift beschreibt die Durchführung der Reaktion in wässriger Lösung, jedoch ist keine Information zu Qualitätsparametern des Dimethylglucamins mit Ausnahme des Schmelzpunktes zu finden. Das bedeutet, dass eine Kristallisation aus der wässrigen Reaktionslösung durchgeführt worden sein muss, die in guten Ausbeuten zum Produkt führt. Dies ist jedoch für die kommerzielle Verwendung des Dimethylglucamines nicht vorteilhaft, da Dimethylglucamin als Feststoff mit einem Schmelzpunkt > 100 °C schwieriger als eine Flüssigkeit zu handhaben ist und es vorteilhaft wäre, Synthesebedingungen zu finden, die direkt ohne einen Aufreinigungsschritt zu einer Lösung von Dimethylgucamin in einem wasserhaltigen Medium führt. In diesem Falle sind neben Ausbeute und Schmelzpunkt des umkristallisierten Dimethylglucamins weitere Qualitätsparameter relevant, wie z. B. die Farbe der Reaktionslösung und der Gehalt an Ausgangsverbindugen wie N-Methylglucamin, Formaldehyd oder Nebenprodukten wie Säuren, die ohne Umkristallisation im Produkt verbleiben und dementsprechend limitiert werden müssen. Auf der anderen Seite ist das beschriebene Verfahren im Großmaßstab nicht vorteilhaft, weil die Hydrierung als exotherme Reaktion nach der Zusammenführung von Polyhydroxyamin und Aldehyd nicht gut zu steuern ist und sicherheitstechnische Probleme aufweist. Andere Verfahren beschreiben zwar ein einstufiges Verfahren, allerdings erfordert z. B. DE-2118283 die Verwendung von Ag-Pd Katalysatoren und ebenfalls hohe Temperaturen von 100 °C bis 250 °C. Die Verwendung eines teuren Katalysators führt zu höheren Produktionskosten.

EP-A-663 389 lehrt ein Verfahren zur Herstellung von Aminoalkoholen, dadurch gekennzeichnet, dass man Hydroxycarbonylverbindungen mit Wasserstoff und Ammoniak oder einem primären oder sekundären Amin bei Temperaturen von 0 bis 300 °C und Drücken von 1 bis 400 bar in Gegenwart von Katalysatoren, deren katalytisch aktive Masse aus 50 bis 100 Gew.-% Ruthenium besteht, umsetzt.

Des Weiteren ist bekannt, dass bei der Herstellung von tertiären Aminen aus sekundären Aminen mit Formaldehyd und Wasserstoff nur schlechte Ausbeuten von unter 90 % erreicht werden können. EP-0142868B1 beschreibt, dass bessere Ergebnisse erreicht werden können, wenn geträgerte Katalysatoren welche maximal 10 wt.-% Ni, Co, Ru, Rh Pd or Pt auf Aktivkohle enthalten verwendet werden. Übliche und günstige Hydrierkatalysatoren wie Raney-Ni oder Raney-Co führen zu schlechteren Produktqualitäten. Auch in diesem Verfahren sind lediglich gaschromatographische Reinheiten bestimmt worden.

Um trotzdem diese üblichen Katalysatoren einzusetzen, wird zum Beispiel in GB-908203 vorgeschlagen, die Reaktion ausgehend von 1,3,4,6-tetraacetyl-D-glucosamine zu starten bzw. wasserentziehende Stoffe wie Zeolithe, MgSO₄ oder CaCl₂ (US-4190601) einzusetzen.

Die bekannten Verfahren bieten somit nur unzureichende Lösungen für die Herstellung von N,N-Dimethylglucamin-Lösungen aus N-Methylglucamin. So können die notwendigen Qualitätsparameter, speziell die Farbe der Reaktionslösung, aber auch der Rest-Nickelgehalt oft nicht eingehalten werden (siehe Vergleichsbeispiele) oder die Ausbeuten sind zu gering. Auch führen die bekannten Verfahren zu höheren Herstellkosten, da sie entweder mehrstufige Synthesen, Aufreinigung der rohen Reaktionslösung oder teurere Ausgangssubstanzen (z. B. 1,3,4,6-tetraacetyl-D-glucosamine oder hochreines N-Methylglucamin) erfordern oder teure Katalysatoren verwendet werden müssen. Ein Recycling der Katalysatoren um Kosten zu sparen ist in den bekannten Verfahren ebenfalls nicht beschrieben.

Daher ist die Bereitstellung eines kostengünstigen, katalytischen Verfahrens zur Herstellung von N,N-Dimethylglucamin-Lösungen ausgehend von kostengünstigem, technischen oder reinem N-Methylglucamin notwendig. Zur Eignung für kommerzielle Anwendungsgebiete wie beispielsweise Farben- und Lackherstellung, Pflanzenschutz, Medizin und Kosmetik muss das nach dem erfindungsgemäßen Verfahren hergestellte N,N-Dimethylglucamin und dessen wässrige Lösungen bestimmte Qualitätsanforderungen in Bezug auf Farbe und maximalen Gehalt an bestimmten Nebenkomponenten erfüllen, die mit den Verfahren des Standes der Technik nicht erreicht werden können.

Es wurde überraschenderweise gefunden, dass bei der Mischung der Ausgangsstoffe Formaldehyd und N-Methylglucamin in Gegenwart von Wasserstoff unter Druck und eines Katalysators ein verbessertes Produkt erhalten wird.

Gegenstand der Erfindung ist ein Verfahren zur Herstellung einer wässrigen N,N-Dimethylglucaminlösung, dadurch gekennzeichnet, dass in Gegenwart eines Metallkatalysators bei 10-200 bar Wasserstoffdruck eine wässrige Lösung von Formaldehyd zu einer wässrigen Lösung von N-Methylglucamin zudosiert wird, wobei im Anschluss an eine Hydrierung unter Formaldehyddosierung bei 35-65°C, ein weiterer Nachhydrierschritt ohne Formaldehyddosierung bei 70 - 110 °C angeschlossen wird.

Ein einstufiges Verfahren ist dadurch gekennzeichnet, dass alle Komponenten gleichzeitig in einem Reaktor ohne zwischenzeitliche Abtrennung oder Aufreinigung umgesetzt werden.

Als Katalysator wird ein nickel-oder cobalthaltiger Katalysator, bevorzugt ein nickelhaltige Katalysator, insbesondere bevorzugt Raney-Nickel verwendet. Des Weiteren ist die Reaktion unter Wasserstoffatmosphäre und Dosierung von Wasserstoff durchzuführen um den Druck im angegebenen Druckbereich konstant zu halten. Die Konzentration der eingesetzten wässrigen N-Methylglucaminlösung liegt vorzugsweise im Bereich von 30 - 70 Gew.-%, bevorzugt 35 - 65 Gew.-%, insbesondere bevorzugt 40 - 60 Gew.-%. Formaldehyd wird als wässrige Lösung bevorzugt mit einer Konzentration zwischen 10 und 60 Gew.-%, insbesondere bevorzugt zwischen 30 und 40 Gew.-% verwendet. Bevorzugt sind hier Formaldehydlösungen, die einen geringen Restgehalt an Methanol enthalten.

Alle Prozentangaben sind Gewichtsprozente, es sei denn eine andere prozentuale Basis wäre angegeben.

Im erfindungsgemäßen Verfahren liegt das Molverhältins N-Methylglucamin : Formaldehyd vorzugsweise bei 1 : 1 bis 1 : 1,5, mehr bevorzugt bei 1 : 1 bis 1 : 1,2, insbesondere bevorzugt bei 1 : 1,01 bis 1 : 1,08.

Der Wasserstoffdruck liegt vorzugsweise bei p = 10 - 200 bar, mehr bevorzugt 20 - 180 bar, insbesondere bevorzugt bei 70 - 120 bar.

Die Reaktionstemperatur liegt im erfindungsgemäßen Verfahren vorzugsweise bei T = 30 - 100 °C, mehr bevorzugt 35 - 65 °C, insbesondere bevorzugt 40 - 50 °C.

Stellt man eine Dimethylglucamin-Lösung mit dem erfindungsgemäßen Verfahren her, so beträgt die Hazen-Farbzahl einer resultierenden, 50 % Lösung von N,N-Dimethylglucamin in Wasser weniger als 800, bevorzugt < 400, insbesondere < 230.

Der Katalystor kann im erfindungsgemäßen Verfahren mehrmals verwendet werden, bevorzugt wird er mehr als 5 x wiederverwendet.

Die nach dem erfindungsgemäßen Verfahren hergestellten Lösungen enthalten, bezogen auf eine 50 %ige Dimethylglucaminlösung, weniger als 2 %, bevorzugt weniger als 1 %, besonders bevorzugt < 0.25 % des Ausgangsstoffs N-Methylglucamin.

Die nach dem erfindungsgemäßen Verfahren hergestellten Lösungen enthalten, bezogen auf eine 50 %ige Dimethylglucaminlösung, bevorzugt < 0.1 % Formaldehyd.

An das erfindungsgemäße Verfahren kann im Anschluss an die Hydrierung ein Destillationsschritt zur Entfernung von überschüssigem Wasser und dem als Nebenprodukt erhaltenen Methanol erfolgen. Hierbei wird ein Restgehalt von bevorzugt < 0.1 % Methanol erhalten.

In einer weiteren Ausführungsform wird im Anschluss an an das erfindungsgemäße Verfahren ein Nachhydrierschritt angeschlossen. Dazu wird nach der vollständigen Zugabe der Formaldehydlösung und beendeter Wasserstoffaufnahme eine Hydrierung bei 60 - 110 °C vorgenommen. Das aus dem erfindungsgemäßen Verfahren erhaltene Produkt wird vor der Nachhydrierung vorzugsweise nicht isoliert.

Das nach dem erfindungsgemäßen Verfahren hergestellte Produkt liefert die folgenden Qualitätsparameter (bezogen auf eine 50 % Lösung von N,N-Dimethlyglucamin in Wasser):
- Die Produkte weisen eine Hazen Farbzahl kleiner 800, bevorzugt kleiner 400; insbesondere kleiner 230 auf. Dies ist zum Beispiel für die Anwendung in der Kosmetik oder in Farben und Lacken entscheidend.
- Der Restgehalt an N-Methylglucamin (Bestimmung mittels GC) ist kleiner 2 Gew.-%, bevorzugt kleiner 1 Gew.-%, insbesondere kleiner 0,25 Gew.-%. Erhöhte Werte würden zu einem höheren Anteil an sekundären Aminen und potentieller Nitrosierbarkeit führen, was für eine Vielzahl an Anwendungen und Formulierungen störend ist.
- Des Weiteren kann die Anwesenheit von kurzkettigen freien sowie chemisch gebundenen Monocarbonsäuren bei verschiedenen Anwendungen durch z. B. Salzbildung stören. Als Leitsubstanz wird hier der Gehalt an Ameisensäure (Bestimmung über lonenchromatographie) beschrieben, welcher mit dem erfindungsgemäßen Verfahren unter 1 Gew.-%, bevorzugt unter 0,5 Gew.-%, insbesondere unter 0,1 Gew.-% liegt.
- Der Gehalt an freiem und chemisch gebundenem Formaldehyd liegt mit dem beschriebenen Verfahren unter 0,5 Gew.-%, bevorzugt kleiner 0,1 Gew.-%, insbesondere kleiner 0,05 Gew.-%.
- Das erfindungsgemäße Verfahren liefert Produkt, welches einen geringen Gehalt an unerwünschten Metallverunreinigungen wie z. B. Aluminium, Cobalt oder Nickel aufweist (Bestimmung über ICP-OES). Der Nickelgehalt ist kleiner 50 ppm, bevorzugt kleiner 20 ppm, insbesondere kleiner 10 ppm.
- Für die Anwendung störend sind ebenfalls flüchtige organische Verbindungen wie z. B. Methanol, dessen Gehalt mit dem beschriebenen Verfahren unter 0,7 Gew.-%, bevorzugt unter 0,5 Gew.-% insbesondere bevorzugt unter 0,1 Gew.-% liegt.

Das erfindungsgemäße Verfahren wird bevorzugt in einem gerührten Reaktor oder in einem Schlaufenreaktor mit externem Umpump und Durchmischung durchgeführt. Dieser Reaktor ist temperiert um gegebenenfalls auftretende Exo- oder Endothermien abfangen zu können und die Temperatur über die gesamte Reaktionszeit konstant zu halten. Während der Reaktion wird Formaldehyd kontinuierlich oder abschnittsweise, bevorzugt kontinuierlich zudosiert und mit oder ohne weitere Reaktionszeit ohne weitere Formaldehydzugabe durchgeführt. Die genaue Reaktionszeit kann über Probenahmen und in Prozess Kontrolle bestimmt werden.

Die genannten Katalysatoren (z. B. Raney-Ni) verbleiben nach Filtration im Reaktor und werden für weitere Synthesen verwendet. Dies bietet einen entscheidenden Vorteil gegenüber anderen Verfahren, bei denen der Katalysator nicht oder nur wenige Male wiederverwendet werden kann was zu einem erheblichen Anstieg der Produktionskosten führt. Eventuell auftretende Verluste an Katalysatormaterial durch die Filtration können vor jedem neuen Ansatz ergänzt werden.

Um eventuell in der Reaktionslösung vorhandene flüchtige Komponenten zu entfernen oder die gewünschte Konzentration an Wasser einzustellen, kann die erhaltene Reaktionslösung mittels einer Strippung oder Destillation oder einem ähnlichen dem Fachmann bekannten Verfahren aufgearbeitet werden. Die Strippung kann unter Zugabe von Stickstoff oder Wasser bei Temperaturen zwischen 20 - 100 °C, bevorzugt 30 - 80 °C, insbesondere bevorzugt zwischen 40 und 60 °C bei dem entsprechenden Dampfdruck von Wasser stattfinden.

Das erfindungsgemäße Verfahren führt somit zu Mischungen von N-Methylglucamin und Wasser im Mengenverhältnis z. B. 1 : 99 bis 99 : 1, bevorzugt 30 : 70 bis 90 : 10, insbesondere bevorzugt 45 : 55 bis 80 : 20.

Falls für die Anwendung des Produktes noch geringere Konzentrationen an Metallen gewünscht werden, kann eine Aufarbeitung über einen lonentauscher oder ein ähnliches dem Fachmann bekanntes Verfahren stattfinden.

Des Weiteren kann die nach dem erfindungsmäßigen Verfahren hergestellte N,N-Dimethylglucaminlösung auch als reines, kristallines N,N-Dimethylglucamin hergestellt werden. Dies kann durch dem Fachmann bekannte Aufarbeitungsverfahren, zum Beispiel Abdestillation des Wassers oder/und Umkristallisation aus verschiedenen Lösungsmitteln wie etwa Alkohol oder Alkohol/Wasser Gemische geschehen, und ist für die Anwendung im Pharma- und Medizinbereich von Bedeutung.

Für das erfindungsgemäße Verfahren werden Paraformaldehyd oder wässrige Formaldehydlösungen eingesetzt. Bevorzugt sind wässrige Formaldehydlösungen mit 30 - 40 % Aktivgehalt, besonders bevorzugt solche, die einen niedrigen Gehalt an Methanol enthalten.

Das verwendete N-Methylglucamin kann als hochreine, umkristallisierte Ware eingesetzt werden, bevorzugt jedoch in technischer Qualität. Technisches N-Methylglucamin kann im Prinzip nach WO-92/06073 aus Glucosesirup hergestellt werden und fällt ohne weitere Aufreinigung als eine etwa 60 % wässrige Lösung an.

Dieses kann durch Umkristallisation weiter aufgereinigt werden. Dies ist jedoch für farblich gutes N,N-Dimethylglucamin nicht notwendig.

Das erfindungsgemäße Verfahren ermöglicht die Produktion von N,N-Dimethylglucamin mit Hilfe eines kostengünstigen und einfachen Verfahrens. Im Vergleich zu bisher bekannten Verfahren (z. B. EP-614881A1) ist das Verfahren leichter zu kontrollieren, führt zu niedrigeren Produktionskosten, besserer Produktqualität und somit zu einem breiteren Anwendungsspektrum.

Die niedrigen Produktionskosten entstehen dadurch, dass der einstufige Prozess keine Isolierung eines Zwischenproduktes erfordert. Im Vergleich zu EP-614881A1 ist die Isolierung des Additionsproduktes vor der Hydrierung nicht erforderlich und somit wird die Anlagenbelegungszeit minimiert. Ebenso führt der verwendete günstige nickelhaltige oder cobalthaltige Katalysator zu einer Reduzierung der Kosten im Vergleich zu anderen Verfahren welche die Verwendung von kostenintensiven Katalysatoren wie Ru, Ag-Pd oder Pt erfordern. Durch die Möglichkeit den Katalysator für viele Reaktionszyklen wiederzuverwenden, werden die Produktionskosten ebenfalls erheblich gesenkt.

Überraschenderweise konnte im Gegensatz zu US-4190601 gefunden werden, dass die Anwesenheit von Wasser die Reaktion nicht stört. Daher kann dieses als Lösemittel verwendet werden. Dies führt bei Einhaltung der beschriebenen, geeigneten Konzentrationen zu zahlreichen Vorteilen bei der Handhabung wie zum Beispiel einer Erniedrigung der Viskosität und der Vermeidung der Kristallisation des Produktes bei Raumtemperatur bzw. Reaktionstemperatur. Ebenso führt die Verwendung von Wasser als Lösemittel zu einer Reduktion der Kosten, Erniedrigung des Gefahrenpotentials und Vermeidung von toxischen bzw. gefährlichen Abfällen.

Die Einhaltung der für die Anwendung erforderlichen Qualitätsparameter ist bei den im Stand der Technik beschriebenen, bekannten Verfahren nicht ausreichend beschrieben bzw. die Verfahren führen zu schlechterer Qualität. Die Vergleichsbeispiele zeigen, dass das erfindungsgemäße Verfahren Vorteile bei den Qualitätsparametern, speziell der Produktfarbe, aufweisen und damit eine aufwändige Aufreinigung vermieden werden kann.

**Beispiele**

| | 1-stufig/ 2-stufig | Hydrierdruck | Hydriertemperatur [°C] | Farbe Gardner bei 50 % Aktivgehalt | Farbe Hazen bei 50 % Aktivgehalt | NMG [%] | Ni [ppm] | Formaldehyd [%] |
|---|---|---|---|---|---|---|---|---|
| 1 (Vgl.) | 2-stufig | 30 | 100 | 9,4 | n.m. | 0,91 | 35 | 0,03 |
| 2 (Vgl.) | 2 -stufig | 100 | 130 | 16,9 | n.m. | 6,0 | 29 | 0,01 |
| 1 | 1 -stufig | 28 | 60 | 2,5 | 350 | 0,06 | 2 | 0,02 |
| 2 | 1-stufig, repeat | 28 | 60 | 1,1 | 200 | 0,08 | 2 | 0,02 |
| 3 | 1-stufig | 100 | 60 | 0,6 | 105 | 0,10 | 2 | 0,02 |
| 4 | 1-stufig, repeat | 100 | 60 | 0,6 | 115 | 0,05 | 3 | 0,01 |
| 3 (Vgl.) | 1-stufig | 3 | 60 | 9 | n.m. | 0,17 | 62 | 0,03 |
| 4 (Vgl.) | 1-stufig | 3 | 60 | 8 | n.m. | 0,9 | 45 | 0,03 |
| 5 (Vgl.) | 1 -stufig | 85 | 40 | 0.5 | 81 | 0,13 | 0,6 | 0,01 |

| | | | | | | | | |
|---|---|---|---|---|---|---|---|---|
| n.m. = nicht messbar | | | | | | | | |

Gardnerfarbzahl und Hazenfarbzahl:
Die klaren und gasblasenfreien, wässrigen Dimethylglucamin-Lösungen wurden in 10 mm-Rechteckküvetten eingefüllt. Die Farbzahlmessungen erfolgten bei Raumtemperatur in einem Farbzahlmessgerät vom Typ LICO 690 der Firma Hach Lange.

Gesamtaminzahl durch Säure-Base-Titration:
Die auf einer Analysenwaage genau eingewogenen Proben wurden in Eisessig gelöst und mit 0,1 molarer Perchlorsäure in Eisessig und einem Titroprozessor der Firma Metrohm titriert.

Trockensubstanz (105 °C/2 Stunden):
Die genau eingewogenen Proben wurden in einem Trockenschrank bei 105 °C zwei Stunden lang durch Abdampfen des Wassers bis zur Gewichtskonstanz getrocknet und nach der Trocknung genau ausgewogen.

Wasser (Karl Fischer-Titration):
Der Wassergehalt der genau eingewogenen Proben wurde nach der Karl Fischer-Titrationsmethode mit Karl Fischer-Solvent und Karl Fischer-Titrant bestimmt.

N-Methylglucamin, N,N-Dimethylglucamin und Sorbitol (GC):
Die Proben wurden mit einem sehr großen Überschuss an Essigsäureanhydrid/Pyridin bei 80 °C vollständig acetyliert. Die gaschromatographische Bestimmung des Gehalts an N-Methylglucamin, N,N-Dimethylglucamin und Sorbitol erfolgte auf einer 60 m-Agilent HP-5-Säule mit Decanol als internem Standard und einem FID-Detektor.

Methanol (GC):
Der Gehalt an Methanol wurde nach einer gaschromatographischen Methode für leicht flüchtige Substanzen mit Isobutanol als internem Standard und WLD-Detektor bestimmt.

Formaldehyd (frei und chemisch gebunden):
Die Proben wurden zur Freisetzung des chemisch gebundenen Formaldehyds mit wässriger Schwefelsäure in Anwesenheit von 2,4-Dinitrophenylhydrazin erhitzt.
Durch die Reaktion des Formaldehyds mit 2,4-Dinitrophenylhydrazin bildete sich 2,4-Dinitrophenylhydrazon. Der Gehalt an 2,4-Dinitrophenylhydrazon wurde anschließend flüssigchromatographisch durch HPLC bestimmt. Der Gehalt an freiem Formaldehyd und chemisch gebundenem Formaldehyd wurde als Summe erfasst.

Nitrosamine (total NNO):
Die Proben wurden auf den Gesamtgehalt an Nitrosaminen in Anlehnung an die Methode ATNC (apparent total nitrosamine content) untersucht. Unter chemischer Denitrosierung im sauren Milieu wurde aus den Nitrosaminen Stickstoffmonoxid freigesetzt und danach mit einem sehr empfindlichen Stickstoffmonoxidspezifischen Detektor quantitativ bestimmt. Die Berechnung und Gehaltsangabe erfolgte als NNO-Gehalt (>N-N=O mit Molmasse 44 g/mol).

Nickel (ICP-OES):
Der Gehalt an Nickel in den Proben wurde mittels ICP-OES (inductively coupled plasma optical emission spectrometry; optische Emissionsspektrometrie mittels induktiv gekoppelten Plasmas) in Anlehnung an DIN EN ISO 11885 bestimmt.

Ameisensäure (frei und chemisch gebunden, IC):
Die Proben wurden im wässrig alkalischen Milieu in der Hitze vorbehandelt, um die als Ester und Amid chemisch gebundene Ameisensäure durch Hydrolyse frei zu setzen. Der Gehalt an bereits vor der alkalischen Hydrolyse vorhandener Ameisensäure oder deren Salze und an Ameisensäure bzw. deren Salze, die erst durch die alkalische Hydrolyse frei gesetzt worden waren, wurde als Summe durch lonenchromatographie (IC) bestimmt.

### Vergleichsbeispiel 1: Beispiel 2 aus EP-0614881

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen N-Methylglucaminlösung (Gardnerfarbzahl 1,7, Hazenfarbzahl 263) hergestellt. Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Die Gardnerfarbzahl der blassgelben, klaren wässrigen N-Methylglucamin-Formaldehyd-Adduktiösung betrug 0,8 und die Hazenfarbzahl 151.

Unmittelbar nach Ende des Zutropfens wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Unter weiterem Rühren wurde auf 100 °C erhitzt und danach Wasserstoff zugeführt. Die exotherme Hydrierung erfolgte unter Nachpressen des verbrauchten Wasserstoffs bei 800 Upm, 100 °C und 30 bar Wasserstoffdruck. Nach Ende der erkennbaren Wasserstoffaufnahme wurde noch zwei Stunden bei 100 °C und 30 bar Wasserstoffdruck gerührt. Danach wurde auf 30 °C abgekühlt, entspannt, mit Stickstoff gespült und der 2 Liter-Rührautoklav entleert. Der Raney-NickelKatalysator wurde durch eine Druckfiltration unter Stickstoff abgetrennt. Das flüssige Filtrat war dunkelbraun und hatte die Gardnerfarbzahl 8.

Dieses dunkelbraune Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51 % Wirkstoff und 49 % VE-Wasser eingestellt. Diese Produktlösung wurde analysiert. Bis auf die Verluste bei der Entleerung und Aufarbeitung war die Ausbeute an gelöstem N,N-Dimethylglucamin im DMG 50 nahezu quantitativ.

Das erhaltene Produkt war wie folgt charakterisiert:

| | |
|---|---|
| Aussehen bei 20 °C: | klar, flüssig, dunkelbraun |
| Gardnerfarbzahl: | 9,4 |
| Hazenfarbzahl: | nicht messbar, da zu dunkel |
| Gesamtaminzahl (Titration): | 133 mg KOH/g |
| Trockensubstanz (105 °C/2 Stunden): | 51,4 % (m/m) |
| Wasser (Karl-Fischer Titration): | 49 % (m/m) |
| N-Methylglucamin (GC): | 0,91 % (m/m) |
| N,N-Dimethylglucamin (GC): | 44,5 % (m/m) |
| Sorbitol (GC): | 0,6 % (m/m) |
| Methanol (GC): | < 0,1 % (m/m) |
| Formaldehyd (frei und chemisch gebunden, LC): | 0,026 % (m/m) |
| Nitrosamine (total NNO): | < 50 µg/kg |
| Nickel (ICP-OES): | 35 µg/g |
| Ameisensäure (frei und chemisch gebunden) | 0,70 % (m/m) |

### Vergleichsbeispiel 2: nach Beispiel 3 aus EP-0614881

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen, technischen N-methylglucaminlösung (Gardnerfarbzahl 1,7, Hazenfarbzahl 263) mit VE-Wasser hergestellt.

Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Danach wurde die klare, blassgelbe Reaktionsmischung zusätzlich noch eine Stunde bei 35 °C gerührt. Die Gardnerfarbzahl dieser blassgelben wässrigen N-Methylglucamin-Formaldehyd-Adduktlösung betrug 0,8 und die Hazenfarbzahl 162.

Danach wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Unter weiterem Rühren wurde auf 125 °C erhitzt und danach Wasserstoff zugeführt. Die exotherme Hydrierung erfolgte unter Nachpressen des verbrauchten Wasserstoffs bei 800 Upm, 130 °C und 100 bar Wasserstoffdruck. Nach Ende der erkennbaren Wasserstoffaufnahme wurde noch zwei Stunden bei 130 °C und 100 bar Wasserstoffdruck gerührt. Danach wurde auf 30 °C abgekühlt, entspannt, mit Stickstoff gespült und der 2 Liter-Rührautoklav entleert. Der Raney-NickelKatalysator wurde durch eine Druckfiltration unter Stickstoff abgetrennt. Das flüssige Filtrat war tief dunkelbraun und hatte die Gardnerfarbzahl 13,4.

Dieses tief dunkelbraune Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51% Wirkstoff und 49 % VE-Wasser eingestellt. Die erhaltene Produktlösung wurde analysiert. Bis auf die Verluste bei der Entleerung und Aufarbeitung war die Ausbeute an gelöstem N,N-Dimethylglucamin im DMG 50 nahezu quantitativ.

Das erhaltene Produkt war wie folgt charakterisiert:

| | |
|---|---|
| Aussehen bei 20 °C: | klar, flüssig, tief dunkelbraun |
| Gardnerfarbzahl: | 16,9 |
| Hazenfarbzahl: | nicht messbar, da zu dunkel |
| Gesamtaminzahl (Titration): | 133 mg KOH/g |
| Trockensubstanz (105 °C/2 Stunden): | 52,6 % (m/m) |
| Wasser (Karl-Fischer Titration): | 49 % (m/m) |
| N-Methylglucamin (GC): | 6,0 % (m/m) |
| N,N-Dimethylglucamin (GC): | 35,9?? % (m/m) |
| Sorbitol (GC): | 0.6 % (m/m) |
| Methanol (GC): | < 0,1 % (m/m) |
| Formaldehyd (frei und chemisch gebunden, LC): | 0,012 % (m/m) |
| Nitrosamine (total NNO): | < 50 µg/kg |
| Nickel (ICP-OES): | 29 µg/g |
| Ameisensäure (frei und chemisch gebunden) | 0,90 % (m/m) |

### Beispiel 1

In einem 10 Liter-Rührautoklav ausgestattet mit Temperaturmessung, Druckmessung, Sicherheitsventil, Begasungsrührer und Tauchrohr wurden unter einem Gegenstrom von Stickstoff 4,95 kg 60 °C heiße, 57,3 % N-Methylglucamin-Lösung in Wasser, Gardnerfarbzahl: 0,9. Hazenfarbzahl: 158 und 200 g wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 28 bar eingestellt. Im Verlauf von 4 Stunden und 10 Minuten wurden 1,245 kg wässrige Formaldehydlösung (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 59 - 64 °C gehalten. Der verbrauchte Wasserstoff wurde durch Zufuhr von frischem Wasserstoff ergänzt und so der Wasserstoffdruck im 10 Liter-Rührautoklav bei 25 - 28 bar gehalten. Nach Ende der Wasserstoffaufnahme wurde noch 3 Stunden lang bei 60 °C und 28 bar gerührt, auf 90 °C hochgeheizt und weitere 3 Stunden bei 90 °C und 28 bar gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickeis verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die unterhalb des Tauchrohrs im 10 Liter-Rührautoklav verbliebene Menge und die Probenahmen war die Ausbeute an gelöstem N,N-Dimethylglucamin nahezu quantitativ.

Das erhaltene Produkt war wie folgt charakterisiert:

| | |
|---|---|
| Aussehen | klar, flüssig, gelb |
| Gardnerfarbzahl: | 2,5 |
| Hazenfarbzahl: | 350 |
| Gesamtaminzahl (Titration): | 129 mg KOH/g |
| Trockensubstanz (105 °C/2 Stunden): | 50,0 % (m/m) |
| Wasser (Karl-Fischer Titration): | 50 % ( m/m) |
| N-Methylglucamin (GC) | 0,06 % (m/m) |
| N,N-Dimethylglucamin (GC): | 43,5 (m/m) |
| Sorbitol (GC): | 1,0 % (m/m) |
| Methanol (GC): | < 0,01 % (m/m) |
| Formaldehyd (frei und chemisch gebunden, LC): | 0,015 % (m/m) |
| Nitrosamine (total NNO): | < 50 µg/kg |
| Nickel (ICP-OES): | 2 µg/g |
| Ameisensäure (frei und chemisch gebunden) (lonenchromatographie nach alkalischer Hydrolyse, | 0,024 % (m/m) IC) |

### Beispiel 2

In dem 10 Liter-Rührautoklav mit wässrigem N,N-Dimethylglucamin und gebrauchtem Raney-Nickel unterhalb des Tauchrohrs aus dem vorherigen Ansatz (Beispiel 1) wurden unter einem Gegenstrom von Stickstoff 4,95 kg 60 °C heiße, flüssiges, 57,3 % N-Methylglucamin-Lösung in Wasser, (Gardnerfarbzahl: 0,9. Hazenfarbzahl: 158) und 30 g frischer, wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 28 bar eingestellt. Im Verlauf von 3 Stunden und 50 Minuten wurden 1,244 kg wässriger Formaldehyd (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 55 - 64 °C gehalten. Der verbrauchte Wasserstoff wurde durch Zufuhr von frischem Wasserstoff ergänzt und so der Wasserstoffdruck im 10 Liter-Rührautoklav bei 25 - 28 bar gehalten. Die kontinuierliche Bildung des N-Methylglucamin/Formaldehyd-Additionsprodukts und dessen kontinuierliche katalytische Hydrierung zum N,N-Dimethylglucamin erfolgten simultan. Nach Ende der Wasserstoffaufnahme wurde noch 3 Stunden lang bei 60 °C und 28 bar gerührt, auf 90 °C hochgeheizt und weitere 3 Stunden bei 90 °C und 28 bar gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickels verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die Probenahmen war die Ausbeute an gelöstem N,N-Dimethylglucamin nahezu quantitativ.

Das erhaltene Produkt war wie folgt charakterisiert:

| | |
|---|---|
| Aussehen | klar, flüssig, gelb |
| Gardnerfarbzahl: | 1,1 |
| Hazenfarbzahl: | 200 |
| Gesamtaminzahl (Titration): | 139 mg KOH/g |
| Trockensubstanz (105 °C/2 Stunden): | 50,1 % (m/m) |
| Wasser (Karl-Fischer Titration): | 49,8 % (m/m) |
| N-Methylglucamin (GC) *: | 0,08 % (m/m) |
| N,N-Dimethylglucamin (GC): | 46,7 % (m/m) |
| Sorbitol (GC): | 1,1 % (m/m) |
| Methanol (GC): | < 0,01 % (m/m) |
| Formaldehyd (frei und chemisch gebunden, LC): | 0,021 % (m/m) |
| Nitrosamine (total NNO): | < 50 µg/kg |
| Nickel (ICP-OES): Ameisensäure (frei und chemisch gebunden) | 2 µg/g |
| (lonenchromatographie nach alkalischer Hydrolyse, IC) | |

### Beispiel 3

In einem 10 Liter-Rührautoklav ausgestattet mit Temperaturmessung, Druckmessung, Sicherheitsventil, Begasungsrührer und Tauchrohr wurden unter einem Gegenstrom von Stickstoff 4,97 kg 60 °C heiße, flüssige, 57,3 % N-Methylglucamin-Lösung in Wasser (Gardnerfarbzahl: 0,9. Hazenfarbzahl: 158) und 200 g wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 90 bar eingestellt. Im Verlauf von 3 Stunden und 15 Minuten wurden 1,249 kg wässriger Formaldehyd (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 55 - 61 °C gehalten. Der verbrauchte Wasserstoff wurde durch Zufuhr von frischem Wasserstoff ergänzt und so der Wasserstoffdruck im 10 Liter-Rührautoklav bei 97 - 101 bar gehalten. Die kontinuierliche Bildung des N-Methylglucamin/Formaldehyd-Additionsprodukts und dessen kontinuierliche katalytische Hydrierung zum N,N-Dimethylglucamin erfolgten simultan. Durch Probenahmen und Analyse dieser Proben mittels GC, NMR und Titration zur Aminzahlbestimmung wurde die Umsetzung verfolgt. Nach Ende der Wasserstoffaufnahme wurde noch 3 Stunden lang bei 60 °C und 100 bar gerührt, auf 90 °C hochgeheizt und weitere 3 Stunden bei 90 °C und 105 bar gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickels verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die unterhalb des Tauchrohrs im 10 Liter-Rührautoklav verbliebene Menge und die Probenahmen war die Ausbeute an gelöstem N,N-Dimethylglucamin nahezu quantitativ.

Das erhaltene Produkt war wie folgt charakterisiert:

| | |
|---|---|
| Aussehen | klar, flüssig, blassgelb |
| Gardnerfarbzahl: | 0,6 |
| Hazenfarbzahl: | 105 |
| Gesamtaminzahl (Titration): | 133,5 mg KOH/g |
| Trockensubstanz (105°C/2 Stunden): | 50,2 |
| Wasser (Karl-Fischer Titration): | 49,6 % (m/m) |
| N-Methylglucamin (GC) | 0,10 % (m/m) |
| N,N-Dimethylglucamin (GC): | 43,4 (m/m) |
| Sorbitol (GC): | 1,1 % (m/m) |
| Methanol (GC): | < 0,01 % (m/m) |
| Formaldehyd (frei und chemisch gebunden, LC): | 0,024 % (m/m) |
| Nitrosamine (total NNO): | < 50 µg/kg |
| Nickel (ICP-OES): | 2 µg/g |
| Ameisensäure (frei und chemisch gebunden) | 0,015 % (m/m), abhängig von der NMG-Qualität |
| (lonenchromatographie nach alkalischer Hydrolyse, IC) | |

### Beispiel 4

In dem 10 Liter-Rührautoklav mit wässrigem N.N-Dimethylglucamin und gebrauchtem Raney-Nickel unterhalb des Tauchrohrs aus dem vorherigen Ansatz (Beispiel 3) wurden unter einem Gegenstrom von Stickstoff 4,98 kg 60 °C heiße, flüssige, 57,3 % N-Methylglucamin-Lösung in Wasser, (Gardnerfarbzahl: 0,9. Hazenfarbzahl: 158) und 30 g frischer, wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 90 bar eingestellt. Im Verlauf von 3 Stunden und 50 Minuten wurden 1,252 kg wässriger Formaldehyd (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 57 - 62 °C gehalten. Der verbrauchte Wasserstoff wurde durch Zufuhr von frischem Wasserstoff ergänzt und so der Wasserstoffdruck im 10 Liter-Rührautoklav bei 96 - 99 bar gehalten. Die kontinuierliche Bildung des N-Methylglucamin/Formaldehyd-Additionsprodukts und dessen kontinuierliche katalytische Hydrierung zum N,N-Dimethylglucamin erfolgten simultan. Durch Probenahmen und Analyse dieser Proben mittels GC, NMR und Titration zur Aminzahlbestimmung wurde die Umsetzung verfolgt. Nach Ende der Wasserstoffaufnahme wurde noch 3 Stunden lang bei 60 °C und 100 bar gerührt, auf 90 °C hochgeheizt und weitere 3 Stunden bei 90 °C und 108 bar gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickels verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die Probenahmen war die Ausbeute an gelöstem N,N-Dimethylglucamin nahezu quantitativ.

Das erhaltene Produkt war wie folgt charakterisiert:

| | |
|---|---|
| Aussehen: | klar, flüssig, blassgelb |
| Gardnerfarbzahl: | 0,6 |
| Hazenfarbzahl: | 115 |
| Gesamtaminzahl (Titration): | 132 mg KOH/g |
| Trockensubstanz (105 °C/2 Stunden): | 49,9 % |
| Wasser (Karl-Fischer Titration): | 50,3 % (m/m) |
| N-Methylglucamin (GC) *: | 0,05 % (m/m) |
| N,N-Dimethylglucamin (GC): | 45,0 (m/m) |
| Sorbitol (GC): | 1,0 % (m/m) |
| Methanol (GC): | 0,01 % (m/m) |
| Formaldehyd (frei und chemisch gebunden, LC): | 0,012 % (m/m) |
| Nitrosamine (total NNO): | < 50 µg/kg, abhängig von der NMG-Qualität |
| Nickel (ICP-OES): | 2 µg/g, abhängig von der NMG-Qualität |
| Ameisensäure (frei und chemisch gebunden) | 0,024 % (m/m), abhängig von der NMG-Qualität |
| (lonenchromatographie nach alkalischer Hydrolyse, | IC) |

### Vergleichsbeispiel 3 (niedriger Wasserstoffdruck)

In einem 10 Liter-Rührautoklav ausgestattet mit Temperaturmessung, Druckmessung, Sicherheitsventil, Begasungsrührer und Tauchrohr wurden unter einem Gegenstrom von Stickstoff 5,024 kg 60 °C heiße, flüssige, 57,3 % N-Methylglucamin-Lösung in Wasser (Gardnerfarbzahl: 0,9. Hazenfarbzahl: 158) und 200 g wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 3 bar absolut eingestellt. Im Verlauf von 2 Stunden und 45 Minuten wurden 1,263 kg wässriger Formaldehyd (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 54 - 63 °C gehalten. Der verbrauchte Wasserstoff wurde durch Zufuhr von frischem Wasserstoff ergänzt und so der Wasserstoffdruck im 10 Liter-Rührautoklav bei 2,9 - 3,2 bar absolut gehalten. Die kontinuierliche Bildung des N-Methylglucamin/Formaldehyd-Additionsprodukts und dessen kontinuierliche katalytische Hydrierung zum N,N-Dimethylglucamin erfolgten simultan. Durch Probenahmen und Analyse dieser Proben mittels GC, NMR und Titration zur Aminzahlbestimmung wurde die Umsetzung verfolgt. Nach Ende der Wasserstoffaufnahme wurde noch 3 Stunden lang bei 60 °C und 3,5 bar absolut gerührt, auf 90 °C hochgeheizt und weitere 3 Stunden bei 90 °C und 3,9 bar absolut gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickels verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die unterhalb des Tauchrohrs im 10 Liter-Rührautoklav verbliebene Menge und die Probenahmen war die Ausbeute an gelöstem N,N-Dimethylglucamin nahezu quantitativ.

Das erhaltene Produkt war wie folgt charakterisiert:

| | |
|---|---|
| Aussehen | klar, flüssig, braun |
| Gardnerfarbzahl: | 9 |
| Hazenfarbzahl: | > 1000 |
| Gesamtaminzahl (Titration): | 3,54 mequ/g |
| Trockensubstanz (105°C/2 Stunden): | 50,3 |
| Wasser (Karl-Fischer Titration): | 49,8 % (m/m) |
| N-Methylglucamin (GC) | 0,17 % (m/m) |
| N,N-Dimethylglucamin (GC): | 44,3 % (m/m) |
| Sorbitol (GC): | 0,8 % (m/m) |
| Methanol (GC): | < 0,01 % (m/m) |
| Formaldehyd (frei und chemisch gebunden, LC): | 0,029 % (m/m) |
| Nitrosamine (total NNO): | < 50 µg/kg |
| Nickel (ICP-OES): | 62 µg/g |
| Ameisensäure (frei und chemisch gebunden) | 0,15 % (m/m) |
| (lonenchromatographie nach alkalischer Hydrolyse, IC) | |

### Vergleichsbeispiel 4 (niedriger Wasserstoffdruck)

In dem 10 Liter-Rührautoklav mit wässrigem N,N-Dimethylglucamin und gebrauchtem Raney-Nickel unterhalb des Tauchrohrs aus dem vorherigen Ansatz (Beispiel 5) wurden unter einem Gegenstrom von Stickstoff 5,026 kg 60 °C heiße, flüssige, 57,3 % N-Methylglucamin-Lösung in Wasser (Gardnerfarbzahl: 0,9. Hazenfarbzahl: 158) und 30 g frischer, wasserfeuchter Raney-Nickel (enthielt 40 % Wasser) vorgelegt. Mit 200 g VE-Wasser wurde nachgespült. Der 10 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung wurde die Rührgeschwindigkeit auf 1100 Upm, die Innentemperatur auf 60 °C und der Wasserstoffdruck auf 3 bar absolut eingestellt. Im Verlauf von 3 Stunden und 30 Minuten wurden 1,264 kg wässriger Formaldehyd (36,8 % Formaldehyd gelöst in Wasser mit Methanol) kontinuierlich mittels einer Pumpe in den 10 Liter-Rührautoklav dosiert. Das Molverhältnis von N-Methylglucamin zum gesamten Formaldehyd nach Ende der Formaldehyddosierung betrug 1,00 zu 1,05. Die Rührgeschwindigkeit wurde bei 1100 Upm und die Innentemperatur bei 56 - 63 °C gehalten. Der verbrauchte Wasserstoff wurde durch Zufuhr von frischem Wasserstoff ergänzt und so der Wasserstoffdruck im 10 Liter-Rührautoklav bei 3,5 - 3,9 bar absolut gehalten. Die kontinuierliche Bildung des N-Methylglucamin/Formaldehyd-Additionsprodukts und dessen kontinuierliche katalytische Hydrierung zum N,N-Dimethylglucamin erfolgten simultan. Durch Probenahmen und Analyse dieser Proben mittels GC, NMR und Titration zur Aminzahlbestimmung wurde die Umsetzung verfolgt. Nach Ende der Wasserstoffaufnahme wurde noch 3 Stunden lang bei 60 °C und 3,9 bar absolut gerührt, auf 90 °C hochgeheizt und weitere 3 Stunden bei 90 °C und 3,8 bar absolut gerührt. Danach wurde auf 30 °C abgekühlt und der Rührer abgestellt. Eine Stunde lang setzte sich der Raney-Nickel ab. Über ein nicht ganz bis zum Boden reichendes Tauchrohr wurde der Großteil des rohen wässrigen N,N-Dimethylglucamins langsam aus dem 10 Liter-Rührautoklav herausgedrückt und von kleinen Anteilen des suspendierten Raney-Nickels durch Druckfiltration abgetrennt. Der Großteil des Raney-Nickels verblieb mit einem kleinen Rest an wässrigem N,N-Dimethylglucamin unterhalb des Tauchrohrs im 10 Liter-Rührautoklav für den nächsten Ansatz.

Das filtrierte, wässrige N,N-Dimethylglucamin wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Bis auf die Probenahmen war die Ausbeute an gelöstem N,N-Dimethylglucamin nahezu quantitativ.

Das erhaltene Produkt war wie folgt charakterisiert:

| Aussehen | klar, flüssig, braun |
|---|---|
| Gardnerfarbzahl: | 8 |
| Hazenfarbzahl: | > 1000 |
| Gesamtaminzahl (Titration): | 3,53 mequ/g |
| Trockensubstanz (105 °C/2 Stunden): | 50,5 % |
| Wasser (Karl-Fischer Titration): | 49,8 % (m/m) |
| N-Methylglucamin (GC) | 0,9 % (m/m) |
| N,N-Dimethylglucamin (GC): | 42,1 % (m/m) |
| Sorbitol (GC): | 0,8 % (m/m) |
| Methanol (GC): | < 0,01 % (m/m) |
| Formaldehyd (frei und chemisch gebunden, LC): | 0,033 % (m/m) |
| Nitrosamine (total NNO): | < 50 µg/kg |
| Nickel (ICP-OES): | 45 µg/g |
| Ameisensäure (frei und chemisch gebunden) | 0,13 % (m/m) |
| (Ionenchromatographie nach alkalischer Hydrolyse, IC) | |

### Beispiel 5

In einen mit Stickstoff inertisierten 50 Liter-Rührautoklav, ausgestattet mit Temperaturmessung, Druckmessung, Sicherheitsventil, Begasungsrührer und Tauchrohr wurde 1,9 kg wasserfeuchter Raney-Nickel (enthielt 40 % Wasser) vorgelegt. Die Stickstoffatmosphäre wurde nun gegen eine Wasserstoffatmosphäre ausgetauscht. Anschließend wurden 20,0 kg 40 °C heiße, 40,0 % N-Methylglucamin-Lösung in Wasser (Gardnerfarbzahl: 1,4. Hazenfarbzahl: 202) in den Reaktor zudosiert. Nach der Dichtigkeitsprüfung wurde der Reaktorrührer in Betrieb genommen, die Innentemperatur auf 40 °C und der Wasserstoffdruck auf 85 bar eingestellt. Im Verlauf von 3 Stunden wurden 3,49 kg wässrige Formaldehydlösung (36,9 % Formaldehyd gelöst in Wasser mit max. 1,5 % Methanol) kontinuierlich mittels einer Pumpe in den 50 Liter-Rührautoklav dosiert. Das Molverhältnis von N-Methylglucamin zum gesamten Formaldehyd nach Ende der Formaldehyddosierung betrug 1,05. Die Rührgeschwindigkeit wurde bei 490 Upm und die Innentemperatur bei 39 - 43 °C gehalten. Der verbrauchte Wasserstoff wurde durch Zufuhr von frischem Wasserstoff ergänzt und so der Wasserstoffdruck im 50 Liter-Rührautoklav bei 80 - 85 bar gehalten.

Nach Ende der Wasserstoffaufnahme begann die Nachreaktion. Dafür wurde 1 Stunde lang von 40 °C und 85 bar unter Rühren auf 90 °C hochgeheizt, 1 lang bei 90 °C weiter gerührt, und 1 Stunde von 90 °C auf 40 °C abgekühlt. Nach Ende der Nachreaktion wurde auf 30 °C abgekühlt und der Rührer abgestellt. Eine Stunde lang setzte sich der Raney-Nickel ab. Anschließend erfolgte eine Druckfiltration bei 4 bar.

Das filtrierte, wässrige N,N-Dimethylglucamin wurde in einem Batch-Verfahren bei 90 °C und einem Druck von 600 mbar andestilliert, bis die DMG-Lösung 50 %ig war (DMG-50). Das Destillat enthielt vor allem Wasser, wenig Methanol und Spuren von anderen Leichtsiedern. Bis auf die Probenahmen war die Ausbeute an gelöstem N,N-Dimethylglucamin nahezu quantitativ.

Das erhaltene Produkt war wie folgt charakterisiert:

| Aussehen | klar, flüssig, blassgelb |
|---|---|
| Gardnerfarbzahl: | 0,5 |
| Hazenfarbzahl: | 81 |
| Gesamtaminzahl (Titration): | 132 mg KOH/g |
| Trockensubstanz (105 °C/2 Stunden): | 50,2 % |
| Wasser (Karl-Fischer Titration): | 50,0 % (m/m) |
| N-Methylglucamin (GC) | 0,13 % (m/m) |
| N,N-Dimethylglucamin (GC): | 45,3 % (m/m) |
| Sorbitol (GC): | 0,7 % (m/m) |
| Methanol (GC): | < 0,01 % (m/m) |
| Formaldehyd (frei und chemisch gebunden, LC): | 0,005 % (m/m) |
| Nitrosamine (total NNO): | < 50 µg/kg |
| Nickel (ICP-OES): | 0,6 µg/g |
| Ameisensäure (frei und chemisch gebunden) | 0,04 % (m/m) |
| (Ionenchromatographie nach alkalischer Hydrolyse, IC) | |

Aus den Beispielen und Vergleichsbeispielen ergibt sich der Einfluss des Hydrierungsdrucks auf die Farbzahl des erhaltenen Produkts. Ein Produkt mit akzeptabler Hazenfarbzahl wird nur im Druckbereich von 10 bis 200 bar Wasserstoffdruck erhalten.

## Patentansprüche

1. Verfahren zur Herstellung einer wässrigen N,N-Dimethylglucaminlösung, **dadurch gekennzeichnet, dass** in Gegenwart eines Metallkatalysators bei 10-200 bar Wasserstoffdruck eine wässrige Lösung von Formaldehyd zu einer wässrigen Lösung von N-Methylglucamin zudosiert wird, wobei im Anschluss an eine Hydrierung unter Formaldehyddosierung bei 35 - 65 °C, ein weiterer Nachhydrierschritt ohne Formaldehyddosierung bei 70 - 110 °C angeschlossen wird.

2. Verfahren nach Anspruch 1, wobei der Metallkatalysator Raney-Nickel ist.

3. Verfahren nach Anspruch 1 und/oder 2, worin das Molverhältnis N-Methylglucamin zu Formaldehyd 1 : 1 bis 1 : 1,5, bevorzugt 1 : 1 bis 1 : 1,2, insbesondere bevorzugt 1 : 1,01 bis 1 : 1,08 ist.

4. Verfahren nach einem oder mehreren der Ansprüche 1 - 3, das bei einem Wasserstoffdruck von 10 - 200 bar, bevorzugt 20 - 180 bar, insbesondere 70 - 120 bar durchgeführt wird.

5. Verfahren nach einem oder mehreren der Ansprüche 1 - 4, bei dem die Reaktionstemperatur T von 40 - 50 °C beträgt.

6. Verfahren nach einem oder mehreren der Ansprüche 1 - 5, wobei die HazenFarbzahl der erhaltenen, 50 Gew.-% Lösung von N,N-Dimethylglucamin in Wasser kleiner als 800, bevorzugt < 400, insbesondere < 230 ist.

7. Verfahren nach einem oder mehreren der Ansprüche 1 - 6, wobei der Hydrierkatalysator für mehr als 5 Reaktionsansätze verwendet wird.

8. Verfahren nach einem oder mehreren der Ansprüche 1 - 7, wobei in einem Rührreaktor oder Loopreaktor umgesetzt wird.

9. Verfahren nach einem oder mehreren der Ansprüche 1 - 8, wobei der Rest an N-Methylglucamingehalt < 2, bevorzugt < 1, insbesondere < 0.25 Gew.-% beträgt.

10. Verfahren nach einem oder mehreren der Ansprüche 1 - 9, wobei der Rest-Formaldehydgehalt < 0.1 Gew.-% beträgt.

11. Verfahren nach einem oder mehreren der Ansprüche 1 - 10, wobei im Anschluss an die Hydrierung ein Destillationsschritt zur Entfernung von Methanol angeschlossen ist.

## Claims

1. A process for preparing an aqueous N,N-dimethylglucamine solution, wherein an aqueous solution of formaldehyde is metered into an aqueous solution of N-methylglucamine in the presence of a metal catalyst at a hydrogen pressure of 10-200 bar, wherein subsequent to a hydrogenation with formaldehyde metering at 35 - 65°C a further, after-hydrogenation step without formaldehyde metering at 70 - 110°C is appended.

2. The process as claimed in claim 1, wherein the metal catalyst is Raney nickel.

3. The process as claimed in claim 1 and/or 2, in which the molar ratio of N-methylglucamine to formaldehyde is 1 : 1 to 1 : 1.5, preferably 1 : 1 to 1 : 1.2, especially preferably 1 : 1.01 to 1 : 1.08.

4. The process as claimed in one or more of claims 1 - 3, which is carried out at a hydrogen pressure of 10 - 200 bar, preferably 20 - 180 bar, more particularly 70 - 120 bar.

5. The process as claimed in one or more of claims 1 - 4, in which the reaction temperature T is from 40 - 50°C.

6. The process as claimed in one or more of claims 1 - 5, wherein the Hazen color number of the resulting 50 wt% solution of N,N-dimethylglucamine in water is less than 800, preferably < 400, more particularly < 230.

7. The process as claimed in one or more of claims 1 - 6, wherein the hydrogenation catalyst is used for more than 5 reaction batches.

8. The process as claimed in one or more of claims 1 - 7, wherein reaction takes place in a stirred reactor or loop reactor.

9. The process as claimed in one or more of claims 1 - 8, wherein the residual N-methylglucamine content is < 2, preferably < 1, more particularly < 0.25 wt%.

10. The process as claimed in one or more of claims 1 - 9, wherein the residual formaldehyde content is < 0.1 wt%.

11. The process as claimed in one or more of claims 1 - 10, wherein subsequent to the hydrogenation a distillation step for removing methanol is appended.

## Revendications

1. Procédé pour la préparation d'une solution aqueuse de N,N-diméthylglucamine, **caractérisé en ce qu'**en présence d'un catalyseur métallique, à une pression d'hydrogène de 10-200 bars, une solution aqueuse de formaldéhyde est dosée dans une solution aqueuse de N-méthylglucamine, une hydrogénation sous dosage de formaldéhyde à 35-65°C étant suivie d'une autre étape de post-hydrogénation sans dosage de formaldéhyde à 70-110°C.

2. Procédé selon la revendication 1, le catalyseur métallique étant du nickel de Raney.

3. Procédé selon la revendication 1 et/ou 2, le rapport molaire de N-méthylglucamine à formaldéhyde étant de 1:1 à 1:1,5, de préférence de 1:1 à 1:1,2, en particulier de préférence de 1:1,01 à 1:1,08.

4. Procédé selon l'une ou plusieurs des revendications 1-3, qui est réalisé à une pression d'hydrogène de 10-200 bars, de préférence de 20-180 bars, en particulier de 70-120 bars.

5. Procédé selon l'une ou plusieurs des revendications 1-4, dans lequel la température de réaction T est de 40-50°C.

6. Procédé selon l'une ou plusieurs des revendications 1-5, l'indice de couleur de Hazen de la solution à 50% de N,N-diméthylglucamine dans l'eau obtenue étant inférieur à 800, de préférence < 400, en particulier < 230.

7. Procédé selon l'une ou plusieurs des revendications 1-6, le catalyseur d'hydrogénation étant utilisé pour plus de 5 charges réactionnelles.

8. Procédé selon l'une ou plusieurs des revendications 1-7, la transformation étant effectuée dans un réacteur agité ou dans un réacteur à boucle.

9. Procédé selon l'une ou plusieurs des revendications 1-8, le reste de la teneur en N-méthylglucamine étant < 2, de préférence < 1, en particulier < 0,25% en poids.

10. Procédé selon l'une ou plusieurs des revendications 1-9, la teneur résiduelle en formaldéhyde étant < 0,1% en poids.

11. Procédé selon l'une ou plusieurs des revendications 1-10, l'hydrogénation étant suivie d'une étape de distillation pour éliminer le méthanol.
